# EUROPEAN PATENT SPECIFICATION

(11) **EP 2 764 361 B1**
(45) Date of publication and mention of the grant of the patent: **16.09.2015**
(21) Application number: 12780679.2
(22) Date of filing: 04.10.2012
(51) Int. Cl.: G01N 33/50

(54) **DIAGNOSTIC TEST COMPRISING INOSITOL FOR THE ASSESSMENT OF THE FERTILISING ABILITY OF HUMAN SPERMATOZOA**
DIAGNOSTISCHER TEST MIT INOSITOL ZUR BEURTEILUNG DER FERTILISATIONSFÄHIGKEIT MENSCHLICHER SPERMATOZOEN
TEST DE DIAGNOSTIC COMPRENANT DE L'INOSITOL POUR L'ESTIMATION DU POUVOIR FÉCONDANT DE SPERMATOZOÏDES HUMAINS

(30) Priority: 04.10.2011 IT FI20110212
(43) Date of publication of application: 13.08.2014
(73) Proprietor: LO. LI. Pharma S.r.l., 00156 Roma (IT)
(72) Inventor: UNFER, Vittorio, 00155 Roma (IT)
(74) Representative: Brighenti, Livio
(86) International application number: PCT/EP2012/069623
(87) International publication number: WO 2013/050472

(56) References cited:
- WO-A1-2006/116034
- WO-A2-03/072707
- COLONE M ET AL: "Inositol activity in oligoasthenoteratospermia--an in vitro study", RIVISTA EUROPEA PERLE SCIENZE MEDICHE E FARMACOLOGICHE // EUROPEAN REVIEW FOR MEDICAL AND PHARMACOLOGICAL SCIENCES // REVUE EUROPEENNE POUR LES SCIENCES MEDICALES ETPHARMACOLOGIQUES, ROME, IT, vol. 14, no. 10, 1 January 2001 (2001-01-01), pages 891-896, XP008136432, ISSN: 0392-291X

## Description

### Field of the invention

The present invention relates to the field of diagnostic tests and, in particular, predictive tests of fertilising ability of human spermatozoa.

### State of the art

It is estimated that infertility affects about 80 million people in the world and that at least one couple in ten is suffering from primary infertility (no children) or secondary (with children).

The causes of infertility are due both to disorders and pathologies of the male or female reproductive apparatus, amplified if they are present in both partners and to environmental factors and lifestyles that affect reproductive health. The decline in sperm concentration, observed in the male worldwide population, was related to the increased release in the environment of polluting substances, such as xeno-estrogens and heavy metals. Furthermore, in industrialised Countries, the changed social conditions and the relative well-being drive new couples to undertake a reproductive course in a progressively more advanced age.

While, from a biological point of view, the period of greatest fertility in women is around 20 years, the training course and the consolidation of the career, often lead women to seek pregnancy at around 33-35 years, an age when the reproductive potential is already in sharp decline. Therefore, even in the absence of specific reproductive dysfunctions, only the temporal displacement of seeking pregnancy is often sufficient to amplify the sub-fertility causes that may already be present within the couple.

*In vivo,* a dramatic reduction in the number of spermatozoa after ejaculation into the vagina is observed. In fact, many remain trapped within the clot which is formed in the vagina, and only those with rapid progressive motility are able of crossing the cervical channel, the uterus and reach, therefore, the utero-tubal junction, which, due to its narrow diameter, is another natural barrier for the ascent of the spermatozoa in the oviduct. The final step in the selection represented by the *zona pellucida* (egg coat), which will allow to penetrate a single spermatozoon into the oocyte, the most competent for fertilisation. The tuba stores the spermatozoa and maintains the fertilising capacity thanks to the adhesion and the capacitation modulates this event.

Over the last 30 years, research in the Reproductive Biology field, provided fundamental knowledge to the understanding of the mechanisms that are on the basis of gamete maturation, fertilisation and embryonic development. This knowledge gave the basis for the development of biotechnologies that are now applied to medically assisted procreation.

The yield of the techniques of medically assisted procreation closely linked to the number and quality of obtained zygotes, which depends on the quality of the gametes used for fertilisation.

Today, the diagnostic criteria, used to assess whether a patient with normal sperm capacity, are mainly based on semen volume, total number and spermatozoa concentration, total motility, sperm morphology and viscosity with a preference always given to the total number of spermatozoa (see e.g. Bedaiwy et al. 2003).

Data in literature have demonstrated that high concentrations of myo-inositol are present in the seminal plasma; in addition, its role in calcium homeostasis is crucial in maintaining an efficient mitochondrial metabolism.

In published Italian patent application RM2010A000300, in the name of the same Applicant, reported as inositol, alone or in combination with other substances, allows improving the qualities of the spermatozoa *in vitro.*

Despite the obvious advantages offered by the solution proposed in the above mentioned application, it is not yet possible, however, to have a predictive test that allows assessing the fertilising ability of the spermatozoa, the opportunity of subjecting the spermatozoa to treatment with inositol as mentioned above and the related methods, and the percentage success of the treatment.

### Summary of the Invention

It is described a diagnostic test wherein inositol is used that allows assessing the fertilising ability of human spermatozoa and establishing a specific therapy for the patient in case it is necessary to increase the viability of the spermatozoa.

### Detailed description of the invention

Surprisingly, it has now been found that inositol, possibly in combination with other substances, can be used in predictive tests of the conception ability and furthermore in predictive tests of a successful treatment with inositol of the spermatozoa to increase their vitality.

In particular, inositol can be used in combination with antioxidants, culture media, or buffer.

According to the present invention, the test is carried out on samples of semen collected from human donors according to the guidelines of the WHO and allowed to liquefy for the necessary time (normally 10'-20'); then:
- spermatozoa motility is measured on an aliquot of the said sample;
- inositol is added in the sample, by incubating for the required time (typically 20'-40';
- an aliquot is sampled from the solution containing inositol and the spermatozoa motility is measured;

The difference in the number of spermatozoa with progressive motility before and after treatment allows to calculate the percentage increase of the spermatozoa with progressive motility due to treatment with inositol, and therefore to establish the following classification of semen:
*Low responders,* when the rate of increase in the number of spermatozoa with progressive motility is between 0 and 30%,
*Medium responders,* when the rate of increase in the number of spermatozoa with progressive motility is between 31 and 60%.;
*High responders,* when the rate of increase in the number of spermatozoa with progressive motility is between 61 and 100 (or higher)%.

In particular, according to the invention, inositol is used at a final concentration comprised between 1 - 50 mg/ml, preferably 0.5-10 mg/ml.

By inositol, it is intended one of the nine different stereoisomers: myo-inositol, D-chiro-inositol, L-chiro-inositol, scyllo-inositol, muco-inositol, neo-inositol, allo-inositol, epi-inositol and cis-inositol; according to a particular embodiment of the invention the inositol used is myo-inositol.

### Example 1:

The sample formed by the semen collected from donors was left to liquefy for 15' and after shaking it (to prevent that the spermatozoa sediment) two 10µl aliquots are taken and it is proceeded to count, which occurs by using a slide and a coverslip slide.

The semen volume and the coverslip size must be standardised, so that the analysis is carried out on a preparation with a fixed depth of 20µm that enables the spermatozoa to move freely.

The 10µl suspension is then positioned, sampled as mentioned above from the resuspended sample, on a slide and is covered with a 22 mm x 22 mm coverslip slide to create an approximately 20µm deep chamber. The weight of the coverslip will spread the sample.

To each aliquot, the slide is examined with a phase contrast microscope with a magnification of x200 or x400.

Approximately 200 spermatozoa per duplicate are assessed in order to define the percentages of the different motility categories.

The two aliquots used in the example had on average a number of motile spermatozoa equal to 60.

To 0.5 ml of the starting sample is added myo-inositol up to a final concentration of 2 mg/ml and left to incubate at 37 °C for 30'.

Two 10µl aliquots are sampled and it is proceeded with a new motile spermatozoa count according with the methods described above.

The two sampled aliquots on average had a number of motile sperms equal to 90. In this example then, in view that:
The number of motile spermatozoa after treatment with myo-inositol (90), less the number of motile spermatozoa before treatment with myo-inositol (60), gives 30, that is 50% more than the starting data, the semen is classified as a Medium responder according to the classification established above.

To validate the test, 50 men were recruited whose semen was analysed twice per month for three months as described in Example 1.

Only those volunteers whose semen had borderline values with respect to the WHO criteria, but still definable as with normal sperm capacity, were enrolled.

In addition, to the recruited volunteers it was asked not to perform any therapy aimed at improving the quality of the semen.

By running then the myo-inositol diagnostic test as described above, three classes of patients were identified, who differed in the entity of the response: Low, Medium, High,

The volunteers then taken into consideration were the Low (n=11) and the High responders (n=15).

After a year of unprotected intercourses, it was seen how: those who were classified as High responders had failed to conceive (0 on 15), while the volunteers classified as Low responders were able to achieve a pregnancy (8 on 11)

It is therefore proven that the test according to the invention can predict the probability of fertilisation of the spermatozoa.

A further advantage of the described test is that it allows, on the basis of the obtained result, to establish a specific therapy for the patient in case it is necessary to increase the vitality of the spermatozoa, for example by applying a treatment as described in the aforementioned Published Italian Patent Application RM2010A000300.

In fact, for example, for a healthy patient who is classified as Low responder it can be decided to use only oral therapy or *in vitro,* while for Medium and High responders it can be decided to combine the therapies.

## Claims

1. Tests for predicting the conception ability of human spermatozoa wherein:
- samples of sperm are collected and liquefied:
- the motility of spermatozoa is measured
- inositol is added to the sample and it is incubated for the time requested;
- the motility of the spermatozoa after treatment with inositol is remeasured;
- the increment in spermatozoa motility is calculated.

2. Test according to claim 1 wherein inositol has a final concentration of 1 - 50 mg/ml, preferably 0,5 10 mg/ml.

3. Test according to claims 1 - 2 wherein the inositol is one of the nine different stereoisomers: myo-inositol, D-chiro-inositol; L-chiro-inositol, scyllo-inositol, muco-inositol, neo-inositol, allo-inositol, epi-inositol and cis-inositol.

4. Test according to claims 1 - 3 wherein said test is used for predicting the success of a human spermatozoa treatment with inositol for increasing their vitality.

5. Use of inositol in a predictive test for the evaluation of the conception ability of human spermatozoa.

6. Use of inositol in a predictive test for the evaluation of the success of a treatment of human spermatozoa with inositol to increase their vitality.

## Patentansprüche

1. Test zur Vorhersage der Empfängnisfähigkeit menschlicher Spermien, wobei:
- Spermaproben gesammelt und verflüssigt werden:
- die Beweglichkeit der Spermien gemessen wird
- Inositol zu der Probe zugegeben und für eine erwünschte Zeit inkubiert wird;
- die Beweglichkeit der Spermien nach der Behandlung mit Inositol erneut gemessen wird;
- der Unterschied in der Beweglichkeit der Spermien berechnet wird.

2. Test nach Anspruch 1, wobei das Inositol eine Endkonzentration von 1 bis 50 mg/ml, bevorzugt von 0,5 10 mg/ml aufweist.

3. Test nach einem der Ansprüche 1 und 2, wobei das Inositol eines der neun unterschiedlichen Stereoisomere myo-Inositol, D-chiro-Inositol, L-chiro-Inositol, scyllo-Inositol, muco-Inositol, neo-Inositol, allo-Inositol, epi-Inositol und cis-Inositol ist.

4. Test nach einem der Ansprüche 1 bis 3, wobei der Test verwendet wird, um den Erfolg der Behandlung menschlicher Spermien mit Inositol zur Steigerung ihrer Vitalität vorherzusagen.

5. Verwendung von Inositol in einem vorhersagenden Test für die Beurteilung der Empfängnisfähigkeit menschlicher Spermien.

6. Verwendung von Inositol in einem vorhersagenden Test für die Beurteilung des Erfolgs einer Behandlung von menschlichen Spermien mit Inositol, um deren Vitalität zu erhöhen.

## Revendications

1. Test prédictif du pouvoir fécondant de spermatozoïdes humains, dans lequel :
- des échantillons de sperme sont recueillis et liquéfiés ;
- la motilité des spermatozoïdes est mesurée ;
- l'inositol est ajouté à l'échantillon et il est incubé pendant le temps nécessaire ;
- la motilité des spermatozoïdes après traitement par l'inositol est de nouveau mesurée ;
- l'incrément de la motilité des spermatozoïdes est calculé.

2. Test selon la revendication 1, dans lequel l'inositol a une concentration finale de 1 à 50 mg/ml, de préférence de 0,5 à 10 mg/ml.

3. Test selon les revendications 1 et 2, dans lequel l'inositol est l'un de neuf différents stéréoisomères : myo-inositol, D-chiro-inositol, L-chiro-inositol, scyllo-inositol, muco-inositol, néo-inositol, allo-inositol, épi-inositol, cis-inositol.

4. Test selon les revendications 1 à 3, dans lequel ledit test est utilisé pour prédire le succès d'un traitement de spermatozoïdes humains par l'inositol pour augmenter leur vitalité.

5. Utilisation d'inositol dans un test prédictif pour l'évaluation du pouvoir fécondant de spermatozoïdes humains.

6. Utilisation d'inositol dans un test prédictif pour l'évaluation du succès d'un traitement de spermatozoïdes humains par l'inositol pour augmenter leur vitalité.
